# EUROPEAN PATENT APPLICATION

(11) **EP 0 969 100 A1**
(43) Date of publication of application: **05.01.2000**
(21) Application number: 98944255.3
(22) Date of filing: 28.09.1998
(51) Int. Cl.: C12Q 1/68, C12Q 1/66, A61K 31/12, A61K 31/505, A61K 45/00, C12N 15/53

(54) **METHOD FOR SCREENING HEAT SHOCK PROTEIN EXPRESSION INDUCTION REGULATORS**

(30) Priority: 29.09.1997 JP 26414997
(71) Applicant: HSP Research Institute, Inc., Chuo-ku, Osaka-shi, Osaka 541-0045 (JP)
(72) Inventor: MORI, Tetsuya, Okayama 711-0903 (JP); MATSUMOTO, Shuji, Kyoto-shi, Kyoto 601-8037 (JP); YANAGI, Hideki, Hyogo 665-0801 (JP); YURA, Takashi, Kyoto 606-8071 (JP)
(74) Representative: VOSSIUS & PARTNER
(86) International application number: JP9804325
(87) International publication number: WO9916903

(57) **Abstract**

A method for screening heat shock protein (HSP) expression induction regulators such as HSP expression induction inhibitors, HSP expression inducers and HSP expression potentiators, which involves the step of using a cell line transformed by a vector comprising a reporter gene ligated to the downstream of an HSP gene promoter to examine the expression of the reporter gene product in the presence of a test compound; the HSP expression induction inhibitors, HSP expression inducers and HSP expression potentiators which can be screened by the above method: and medicinal compositions containing these HSP expression induction inhibitors, HSP expression inducers or HSP expression potentiators.

## Description

### TECHNICAL FIELD

The present invention relates to a method for screening a substance for controlling induction of expression of heat shock proteins, a substance for controlling induction of expression of heat shock proteins, the substance obtainable by the above method, and a pharmaceutical composition comprising the substance for controlling induction of expression of heat shock proteins.

### BACKGROUND ART

Heat shock proteins (HSPs) have been originally identified as proteins induced when cells are exposed to such stresses as high temperatures, and they may be also induced by stresses other than high temperatures, including, exposure to, for instance, radiation, chemical substances, heavy metals, hypoxia, and the like. A large number of genes encoding HSPs have been known, including the HSP of which expression is induced when a stress such as high temperatures is applied, and the HSP which is expressed homeostatically on a certain level. This presumably means that the HSPs play an important role even under usual conditions, and the expression is induced by applying stress because these proteins are temporarily particularly essential during stress.

The major HSPs can be roughly classified into four families from their molecular weights: HSP90 family, HSP70 family, HSP60 family, and low molecular HSP family.

The functions of the HSPs have long been unknown even though their relevance with various biological functions have been remarked. Recently, unified understanding of their molecular mechanisms has been at last made. In other words, there have been clarified that particularly the HSP70 family and the HSP60 family associate with other polypeptides to play a function of so-called a molecular chaperone (chaperone molecule), which is involved in correct folding (formation of higher order structure), association with a third protein or a nucleic acid (formation of complexes), or localization and membrane permeability within the cell [Hendrick, J. P. and Hartl, F.-U. (1993) *Ann. Rev. Biochem.* **62**, 349-384; Georgopoulos, C. and Welch, W. J. (1993) *Ann. Rev. Cell Biol.* **9**, 601-634].

On the other hand, it has become evident that the HSP has been involved in various diseases which can be stresses to cells during the course of the investigation of HSP mechanism.

While radiotherapy is considered to contribute greatly to cancer therapy, no significant advances were made recently, and there arises a problem that cancer cells tend to be resistant to radiation. Also, hyperthermia is one of auxiliary therapy for cancer therapy, warming of a patient's body may be accompanied by pain, and there arises a similar problem that cancer cells acquire thermotolerance. A major mechanism by which cancer cells acquire resistance to radiation or hyperthermia is considered to be induction of HSP expression in cancer cells in response to the radiation and the hyperthermia which leads to a protective action of the induced HSP on the cancer cells.

There have been known that the induction of expression of 70 kDa and 87 kDa HSPs by heat treatment or by factors other than heat such as arsenic and ethanol are well correlated with the acquisition of thermotolerance of the cells [Li, G. C. and Werb, Z. (1982) *Proc. Natl. Acad. Sci. USA* **79**, 3218-3222]; that a mutant cell line which is resistant to heat has a high level of expression of the HSP70 as compared to the parent strain [Laszlo, A. and Li, G. C. (1985) *Poc. Natl. Acad. Sci. USA* **82**, 8029-8033]; that even when the expression of HSP70 is constitutively enhanced by introducing an HSP70 gene into the cells, the resulting cells are heat resistant as compared to the parent strain [Li, G. C. et al. (1991) *Proc. Natl. Acad. Sci. USA* **88**, 1681-1685]; and that when the expression of HSP70 is conversely inhibited by injecting an antibody against HSP70 to the cells, the resulting injected cells are extremely weak against heat [Riabowol, K. T. et al. (1988) *Science* **242**, 433-436; Johnson, R. N. et al. (1988) *Science* **242**, 1551-1554]. All of these publications suggest the relevancy of HSP70 and the acquisition of thermotolerance.

In addition, cancer cells often express HSP70 at a high level, and a treatment with an antisense oligonucleotide of HSP70 has been known to inhibit proliferation and induce apoptosis [Wei, Y.-q. et al. (1995) *Cancer Immunol. Immunother.* **40**, 73-78].

When the relationship between a cancer and HSPs discussed above and a role played by HSPs expected in a cancer cell is remarked, by the inhibition of the expression-induction of HSP70 in cancer cells, the acquisition of resistance to heat or radiation can be suppressed, and the cell death due to heat or radiation can be increased, so that it is expected that the expression-induction inhibitor of HSP70 is useful as a carcinostatic agent.

Further, P-glycoprotein, which causes a multiple drug resistance of cancer cells against carcinostatic agents, is encoded by an MDR1 gene, and has been known to be induced by heat shock and heavy metals, similarly to HSP [Chin, K. V. et al. (1990) *J. Biol. Chem.* **265**, 221-226; Miyazaki, M. et al. (1992) *Biochem. Biophys. Res. Comm.* **187**, 677-684]. There has been reported that quercetin (discussed later) which inhibits the induction of HSP expression inhibits the induction of P-glycoprotein expression in human cancer cells [Kioka, N. et. al. (1992) *EFBS Lett.* **301**, 307-309], so that it is expected that an HSP expression-induction inhibitor has an inhibitory activity for P-glycoprotein expression-induction and can be useful as an agent for overcoming resistance in cancer chemotherapy.

When neuronal cells are damaged as a result of cerebral ischemia, it undergoes a stress response in a manner similar to that observed in general cells. The induction of HSP70 by global cerebral ischemia has been revealed first by an experiment with gerbils. Specifically, there has been reported that HSP70 is expressed intensively in the cells exposed to a metabolic stress due to ischemia, especially in hippocampal dentate granule cells and in CA3 pyramidal cells, while no expression is observed in hippocampal CA1 pyramidal cells for which the stress is too high, leading to death of the cells [Vass, K. et al., (1988) *Acta Neuropathol.* **77**, 128-135]. Nevertheless, on an mRNA level, an intense expression of HSP70 mRNA has been observed throughout the entire region of hippocampus [Nowak, T. S. (1991), *J. Cereb. Blood Flow Metab.* **11**, 432-439]. On the other hand, there has been reported that when ischemia is loaded for a short time period, the expression of HSP70 is intensely induced in hippocampal CA1 pyramidal cells, and even when severe ischemia is loaded during this period, neuronal cells acquire resistance to the ischemia and survive [Kirino, T. et al., (1991), *J. Cereb. Blood Flow Metab.* **11**, 299-307]. Similar behavior has been observed in rats as well as in gerbils [Nishi, S. et al., (1993) *Brain Res.* **615**, 281-288; Higashi, T. et al., (1994) *Brain Res.* **650**, 239-248; Gaspary, H. et al., (1995) *Mol. Brain Res.* **34**, 327-332].

An involvement of HSP70 in resistance to ischemia is supported by an experiment on a cell level. For example, there have been known that HSP70 overexpressed by introduction of the gene in mouse astrocytes causes resistance to the deficiency of oxygen and glucose which corresponds to *in vitro* ischemia [Papadopoulos, M. C. et al., (1996) *NeuroReport* **7**, 429-432], and that the overexpression in dorsal root ganglia neurons also causes resistance to ischemia [Amin, V. et al., (1996) *Neurosci. Lett.* **206**, 45-48].

Also, in myocardial ischemia, the HSP70 has been known to have a protective action. For example, there has been reported that in a rat treated with heat, the induction of HSP70 in a myocardium is observed, and the size infarct after coronary artery occlusion is significantly reduced [Hutter, M. M. et al., (1994) *Circulation* **89**, 355-360]. The HSP70 overexpression in HSP70 transgenic mice reduces post-ischemic reperfusion-induced myocardial dysfunctions and infarct sizes [Marber, M. S. et al., (1995) *J. Clin. Invest.* **95**, 1446-1456; Plumier, J.-C. L. et al., (1995) *J. Clin. Invest.* **95**, 1854-1860; Radford, N. B. et al., (1996) *Proc. Natl. Acad. Sci. USA* **93**, 2339-2342; Hutte, J. J. (1996) *Circulation* **94**, 1408-1411]. Also in rats to which an HSP70 gene is introduced via an HVJ-liposome, there has been confirmed a protective effect on post-ischemic reperfusion-induced dysfunctions [Suzuki, K. et al., (1997) *J. Clin. Invest.* **99**, 1645-1650].

Furthermore, while there has been reported that the HSP70 is highly expressed in curable wound, almost no or only a low level of expression of HSP70 is found in a chronic wound tissue [Oberringer, M. et al., (1995) *Biochem. Biophys. Res. Commun.* **214**, 1009-1014]. In a skin flap implantation in rats, the flaps isolated from the rats in which HSP70 has been induced previously by means of a heat treatment exhibited a significantly higher survival rate [Koening, W. J. et al., (1992) *Plast. Reconstr. Surg.* **90**, 659-664].

Finally, there has been reported that an antiulcer agent geranylgeranylacetone induces the expression of HSP60 and HSP90 in addition to HSP70 in cultured rat gastric mucosal cells, thereby resulting in exhibiting of a protective action on ethanol-induced injury and exfollation [Hirakawa, T. et al., (1996) *Gastroenterology* **111**, 345-357].

Based on the findings discussed above, it is expected that a pharmaceutical agent which positively induces the HSP can serve as a therapeutic agent or a prophylactic agent against ischemic disease, and also as a wound healing drug or an anti-ulcer agent.

However, extensive studies on the pharmaceutical agents which inhibit or promote the induction of HSP expression cannot be yet said to be made so far. For example, there have been reported as HSP expression-induction inhibitors a plant flavonoid such as quercetin has an inhibitory effect on the HSP expression-induction in human cells [Hosokawa, N. et al., (1990) *Cell Struct. Funct.* **15**, 393-401; Hosokawa, N. et al., (1992) *Mol. Cell. Biol.* **12**, 3490-3498]. Thereafter, there has been reported H-7 and staurosporin which are protein kinase C inhibitors inhibit the HSP expression-induction [Kim, R. S. et al., (1993) *Biochem. Biophys. Res. Comm.* **193**, 759-763; Lee, Y. J. et al., (1994) *Biochem. Pharmacol.* **48**, 2057-2063]. However, these inhibitors are considered to have the effects on various aspects of cellular functions due to the inhibitory effects on protein kinase C, and they are not considered to be pharmaceutical agents having an inhibitory effect specifically to the HSP expression-induction.

Further, as other known pharmaceutical agents for inducing HSP expression, there have been known so far prostaglandin A1 [Amici, C. et al., (1992*) Proc. Natl. Acad. Sci. USA* **89**, 6227-6231], arachidonic acid [Jurivich, D. A. et al., (1994*) Proc. Natl. Acad. Sci. USA* **91**, 2280-2284; Hegde, R. S. et al., (1995) *J. Cell. Physiol.* **165**, 186-200], herbimycin A [Morris, S. D. et al., (1996) *J. Clin. Invest.* **97**, 706-712], aspirin [Amici, C. et al., (1995) *Cancer Res.* **55**, 4452-4457] and the like. However, none of these pharmaceutical agents are considered to have an enhancing action specifically to the HSP expression-induction. Recently, there has been reported that geranylgeranylacetone also has an HSP expression-induction activity [Hirakawa, T. et al., (1996) *Gastroenterol.* **111**, 345-357]. However, a high concentration is required for exhibiting the activity.

In addition, there has not been yet known a method for efficiently screening an expression-induction controlling substance which inhibits or enhances the induction of expression of heat shock proteins.

### DISCLOSURE OF INVENTION

The present invention has been found in view of the above routine techniques, and an object of the present invention is to provide a method of screening a substance for controlling induction of expression, such as an expression-induction inhibitor, an expression inducer, and an expression-induction enhancer of heat shock proteins. Another object of the present invention is to provide an expression-induction inhibitor of heat shock proteins, which is obtainable by such a screening method, comprising as an active ingredient a substance having an inhibitory activity specific to induction of expression of heat shock proteins. Another object of the present invention is to provide an expression inducer of heat shock proteins, which is obtainable by such a screening method, comprising as an active ingredient a substance having an inducing activity specific to induction of expression of heat shock proteins. Another object of the present invention is to provide an expression-induction enhancer of heat shock proteins, which is obtainable by such a screening method, comprising as an active ingredient a substance having an enhancing activity specific to induction of expression of heat shock proteins. Still another object of the present invention is to provide a pharmaceutical composition comprising such an expression-induction inhibitor of heat shock proteins. Still another object of the present invention is to provide a pharmaceutical composition comprising such an expression inducer of heat shock proteins. Still another object of the present invention is to provide a pharmaceutical composition comprising such an expression-induction enhancer of heat shock proteins.

Accordingly, in sum, the present invention pertains to:
[1] a method of screening a substance for controlling induction of expression of a heat shock protein, comprising the step of examining expression of reporter gene products in the presence of a compound to be tested by using a cell line transformed with a vector in which a reporter gene is ligated to downstream of a promoter of a heat shock protein gene;
[2] an expression-induction inhibitor of a heat shock protein obtainable by a screening method comprising the step of examining inhibition of expression of reporter gene products in the presence of a compound to be tested by using a cell line transformed with a vector in which a reporter gene is ligated to downstream of a promoter of a heat shock protein gene;
[3] an expression inducer of a heat shock protein obtainable by a screening method comprising the step of examining induction of expression of reporter gene products in the presence of a compound to be tested by using a cell line transformed with a vector in which a reporter gene is ligated to downstream of a promoter of a heat shock protein gene;
[4] an expression-induction enhancer of a heat shock protein obtainable by a screening method comprising examining enhancement of expression of reporter gene products in the presence of a compound to be tested by using a cell line transformed with a vector in which a reporter gene is ligated to downstream of a promoter of a heat shock protein gene;
[5] a pharmaceutical composition for the treatment of cancer, comprising the expression-induction inhibitor of a heat shock protein according to item [2] above as an active ingredient;
[6] a pharmaceutical composition for the treatment of ischemic disease, wound or ulcer, comprising the expression inducer of a heat shock protein according to item [3] above as an active ingredient; and
[7] a pharmaceutical composition for the treatment of ischemic disease, wound or ulcer, comprising the enhancer for expression-induction of a heat shock protein according to item [4] above as an active ingredient.

### BRIEF DESCRIPTION OF DRAWINGS

Figure 1 is a graphic representation of electrophoresis showing an action of SR-1040 on induction of expression of endogenous HSP70 by means of Northern blotting.
Figure 2 is a graphic representation of electrophoresis showing an action of SR-1040 on induction of expression of endogenous HSP70 by means of Western blotting.
Figure 3 is a graphic representation of electrophoresis showing an action of SR-2125 on induction of expression of endogenous HSP70 by means of Western blotting.
Figure 4 is a graph showing proliferation potencies of cells when lethal heat shock is applied to the cells at 45°C, using BrdU incorporation as an index.
Figure 5 is a graph showing that SR-2297 induces expression-induction of a reporter gene. In the figure, HS(-) means no heat shock is applied to the cells.
Figure 6 is a graph showing that SR-2297 enhances expression-induction of a reporter gene by heat shock. In the figure, HS(+) means heat shock is applied to the cells.
Figure 7 is a graph showing that SR-2297 induces expression of endogenous HSP70. In the figure, HS(-) means no heat shock is applied to the cells.
Figure 8 is a graph showing that SR-2297 enhances expression-induction of endogenous HSP70 by heat shock. In the figure, HS(+) means heat shock is applied to the cells.

### BEST MODE FOR CARRYING OUT THE INVENTION

The present invention provides a method of screening, comprising screening a substance for controlling induction of expression of heat shock proteins by examining the expression of a reporter gene product in the presence of a compound to be tested using a cell line transformed with a vector, the vector resulting from ligating the reporter gene to downstream of a promoter of a heat shock protein gene.

The method of screening of the present invention is a method whereby an expression-induction inhibitor of heat shock proteins, and an expression inducer and expression-induction enhancer of heat shock proteins can be easily screened. That is, in the present specification, the term "a substance for controlling induction of expression of heat shock proteins" intends to encompass an expression-induction inhibitor, an expression inducer and an expression-induction enhancer.

In the present invention, a heat shock protein (HSP) may be any protein as long as expression is induced by exposure to stress, such as heat, radiation, chemical substances, heavy metals and hypoxia. Such HSP includes HSP110 (HSP105), HSP90, HSP70, HSP60, HSP56, HSP47, HSP40, HSP27 and the like.

A heat shock protein gene which is used when the screening method is actually carried out is preferably an HSP gene having a molecular weight of 70 kilo daltons, especially human HSP70 gene. An amino acid sequence and the corresponding nucleotide sequence of human HSP70 are disclosed in Hunt, C. & Morimoto, R. I. (1985) *Proc. Natl. Acad. Sci. USA,* **82**, 6455-6459.

The reporter gene used includes luciferase gene, chloramphenicol acetyltransferase gene, and in view of sensitivity, quantitativeness, operational simplicity and rapidness, the luciferase gene is preferred.

The luciferase gene is contained in, for instance, known vectors, such as pGL2-Basic (manufactured by Promega), and the gene can be used by appropriately cutting off from the above vector by using restriction enzymes and the like.

A method for preparing a vector comprising ligating a reporter gene, such as luciferase gene, to downstream of a promoter of a heat shock protein gene can be carried out by conventional methods. There can be employed, for instance, methods described in Examples set forth below.

The cell line to be transformed includes HeLa cells (ATCC CCL2), BALB/3T3 cells (ATCC CCL163) and CHO cells (ATCC CCL61) and the like, but is not particularly limited thereto. HeLa cells are preferably used in view that they are human cells which are most widely used in studies for heat shock response.

A method of transforming the above cell line by using the above vector includes known methods, such as calcium phosphate method, lipofection method and electroporation method.

The method of screening of the present invention comprises the following steps:
(a) inoculating cells to a plate for culture, the cells transformed with a vector resulting from ligating a reporter gene to downstream of a promoter of a heat shock protein gene, and culturing under an appropriate culture condition, for instance, under a usual culture condition at 35° to 37°C, in a cell medium suitable for culturing the above cells;
(b) adding a compound to be tested to the cell medium of step (a) and culturing;
   wherein there are the following two embodiments as a method of screening an expression-induction inhibitor and an expression-induction enhancer of heat shock proteins
(b-1), and a method of screening an expression inducer of heat shock proteins (b-2):
(b-1) adding a compound to be tested to the cell medium of step (a), thereafter appropriately culturing the cell medium under a condition suitable for culturing the cells, for instance, at 35° to 37°C (e.g. for 0 to 12 hours), subsequently applying heat shock to the cells under a condition suitable for heat shock, for instance, at 40° to 45°C for several hours (e.g. for 10 minutes to 5 hours), and then recovery-culturing under a condition suitable for culturing the cells, for instance, at 35° to 37°C for about 10 minutes to about 12 hours; or
(b-2) adding a compound to be tested to the cell medium of step (a), and culturing under a condition suitable for culturing the cells, for instance, at 35° to 37°C for about 20 minutes to about 48 hours; and
(c) detecting expression of a reporter gene product in the cells of step (b), using expression of a reporter gene product in the cells without addition of a compound to be tested as a control, by means of a method depending upon the reporter gene product.

As described herein, an expression-induction inhibitor and an expression-induction enhancer of heat shock proteins are defined as a compound capable of inhibiting or enhancing expression of the reporter gene product with heat shock. An expression inducer of heat shock protein is defined as a compound capable of inducing expression of the reporter gene product without heat shock.

One example of a method of screening a substance for controlling induction of expression using a luciferase gene as a promoter gene and a reporter gene for the HSP70 gene and HeLa cells will be described concretely below.

### (1) Construction of Plasmids

A reporter plasmid pHBCAT [Wu, B. et al. (1985), *Mol. Cell Biol.* **5**, 330-341; Wu, B. et al. (1986) *Proc. Natl. Acad. Sci. USA* **83**, 629-633] in which 5'-upstream region 2.8 kb of a human HSP70 gene is ligated to a chloramphenicol acetyl transferase (CAT) gene is digested with *Bam*HI. The digested product is then subjected to agarose gel electrophoresis, and a 2.8 kb band is extracted from the gel, to prepare a *Bam*HI fragment containing the 5'-upstream region of the human HSP70 gene. The fragment thus obtained is inserted into the *Bgl*II site of pGL2-Basic (manufactured by Promega) to prepare a reporter plasmid pH70LUC-85 in which a firefly luciferase gene is ligated to the 5'-upstream region, 2.8 kb, of the human HSP70 gene.

In order to examine whether or not luciferase is induced from the reporter plasmid pH707LUC-85 described above in response to heat shock, the induction of luciferase is confirmed in a transient expression system using BeLa cells. Specifically, HeLa cells are inoculated in a culture dish and cultured in Dulbecco's modified Eagle's medium (DMEM) containing 10% fetal calf serum (FCS) for 24 hours, and thereafter transformed with the above pH70LUC-85 and pSVβ-Galactosidase (manufactured by Promega) by a DEAE-dextran method. After culturing at 37°C for 48 hours, heat shock at 41° or 42°C for 2 hours is applied to the resulting cells, followed by recovery-culturing at 37°C for 2 hours. After washing the surface of the cells with PBS, the cells are scraped using a cell scraper. The scraped cells are collected by centrifugation, and the luciferase activity is examined in the cell extract according to the protocol of luciferase assay system (manufactured by Promega) with a luminometer ("LUMINOSKAN," manufactured by Labsystems).

It is found that luciferase activity is detected in the cell extract with heat shock at 41° or 42°C, while the control cell extract without applying heat shock detects no luciferase activity, indicating that luciferase is induced from pH70LUC-85 in response to the heat shock.

Subsequently, a neomycin resistant gene (*neo*) is incorporated into the pH70LUC-85 described above. Specifically, pBluescript-*pgk-neo* in which a *pgk-neo* cassette [McBurney, M. W. et al., (1991) *Nucleic Acids Res.* **20**, 5755-5761] is inserted into the *Eco*RI-*Hind*III site in pBluescript IISK+ (manufactured by Stratagene) is digested with *Not*I and ligated to an *Sal*I-*Not*I adapter. Thereafter, the ligated product is digested with *Sal*I, to prepare a 2 kb *pgk-neo* fragment of which both terminals are *Sal*I cohesive ends. The *pgk-neo* fragment thus obtained is ligated to the *Sal*I site just downstream of the 3'-non-translational region of the luciferase gene in pH70LUC-85, to prepare a reporter plasmid pHLUC-*neo* harboring a neomycin resistant gene.

### (2) Establishment of Cell Lines

Using pHLUC-*neo* prepared in (1), the HeLa cells are transformed by a conventional electroporation method. The transformed HeLa cells are cultured in DMEM containing 700 µg/ml of G-418 (manufactured by Life Technologies) and 10% FCS, whereby selecting G-418 resistant transformants. The transformants thus obtained are grown, and the heat shock-dependent luciferase activities are examined at 90% confluent in 10 cm culture dishes.

Specifically, heat shock at 42°C for 2 hours is applied to the G-418 resistant transformants described above, and then subjected to recovering culture at 37°C for 2 hours. Thereafter, the luciferase activities in cell extracts are determined with the luminometer.

When 30 clones of the G-418 resistant transformants are examined for the luciferase activity, the induction of the heat shock-dependent luciferase activity is observed in 6 clones as described in Example 2 set forth below.

Among these clones, the HeLa-198 strain which shows high fold induction of luciferase by heat shock and gives a high level of expression to an extent sufficient to detect the luciferase activity even in the assay system using a 96-well culture plate is considered to be most suitable to the screening for a substance for controlling HSP70 expression-induction of the present invention.

### (3) Responsiveness of HeLa-198 Strain to HSP70 Expression Inducer

Using quercetin, known to have an inhibitory activity of HSP70 expression-induction, there is examined whether or not the induction of the heat shock-dependent luciferase in the HeLa-198 strain is inhibited. The HeLa-198 strain is inoculated in a 96-well culture plate and cultured in DMEM with a 5%-CO₂ atmosphere at 37°C for 48 hours. Thereafter, quercetin (manufactured by Sigma) is added so as to have a final concentration of 50 to 200 µM, and then the culture is continued for additional 1 hour at 37°C. Subsequently, the heat shock at 42°C for 2 hours is applied, and then the recovering culture is performed at 37°C for 2 hours. As a result of determining the luciferase activity of these cells, it is found that the heat shock-induced luciferase activity is suppressed by quercetin at 50 µM or higher. It is confirmed, for instance, by MTT [3-(4,5-dimethylthiazol-2-yl)-2,5-diphenyltetrazolium bromide] assay using Cell Proliferation Kit (manufactured by Boehringer Mannheim) that this effect does not result from any damages of the cells by the action of quercetin. In addition, quercetin has no effect on expression of luciferase on a background level when no heat shock is applied.

It is found from the results described above that the HeLa-198 strain can be used for the screening for an HSP70 expression-induction inhibitor.

### (4) Responsiveness of HeLa-198 Strain to HSP70 Expression Inducer

Prostaglandin A1 (PGA1) has been known to have an HSP70 expression-induction activity [Amici, C. et al. (1992*) Proc. Natl. Acad. Sci. USA* **91**, 2280-2284]. To a culture of the HeLa-198 strain is supplemented 50 µM of PGA1 (manufactured by Wako Pure Chemical Industries, Ltd.) and cultured for 5 hours, and thereafter the luciferase activity in the cell extract is then examined. As a result, the luciferase activity is revealed to be higher by two to three times when compared to the control without addition of PGA1. From the above, it is revealed that the HeLa-198 strain can be used for the screening for an HSP70 expression inducer.

### (5) Method of Screening Substance for Controlling HSP70 Expression-Induction

The HeLa-198 strain is inoculated in a 96-well culture plate and cultured in DMEM at 37°C under a 5%-CO₂ atmosphere for 48 hours. The culture medium is replaced with DMEM containing a compound to be tested dissolved in DMSO so as to have a final concentration of 10 to 50 µM, and the culture is carried out at 37°C for additional 1 hour. Subsequently, the heat shock at 42°C for 2 hours is applied, and the recovering culture is then performed at 37°C for 2 hours. Here, a system where the culture is continued at 37°C without applying heat shock at 42°C is referred to as a control. After removal of the medium, the luciferase activity in the cell extract is determined with a luminometer ("LUMINOSKAN," manufactured by Labsystems) according to the protocol of Luciferase Assay System (manufactured by Promega).

Using as a control the expression-induction of luciferase in the cells treated with DMEM containing only the solvent DMSO, a compound capable of inhibiting expression-induction of luciferase by heat shock is defined as an expression-induction inhibitor of the HSP70; a compound capable of inducing expression of luciferase at 37°C is defined as an expression inducer of the HSP70; and a compound capable of enhancing expression-induction of luciferase by heat shock is defined as an expression-induction enhancer of the HSP70.

An SV40 early promoter is selected in order to examine whether or not the compound to be tested as described above serves to act on the transcription from a promoter other than that of HSP70. First, the *pgk-neo* cassette described above is incorporated into the *Sal*I site of a luciferase expression plasmid PGL2-Control (manufactured by Promega) utilizing the SV-40 early promoter to prepare pSVLUC-*neo*. Subsequently, pSVLUC-*neo* is used to transform HeLa cells in the same manner as above, and a cell line HeLa-SV-3, which expresses luciferase under the control of the SV40 early promoter, is established.

To a culture of the HeLa-SV-3 cells is supplemented the above compound to be tested (final concentration: 50 µM) and cultured for additional 5 hours, and the luciferase activity in the cell extract is examined. It is defined that the compound to be tested is those which are HSP70 promoter-specific, in case where no significant difference is found as compared to the control with addition of only the solvent DMSO.

The action of the compound to be tested on the induction of expression of endogenous HSP70 can be studied on the mRNA level and the protein level by means of Northern blotting and Western blotting, respectively, as described in Example 6.

Therefore, as described above, the screening method is an epoch-making method capable of carrying out simultaneous and easy screening for an expression-induction inhibitor, an expression inducer and an expression-induction enhancer of heat shock proteins, as well as a method capable of carrying out simultaneous screening of many-compounds to be tested.

In addition, the present invention provides an expression-induction inhibitor of HSPs, which can be obtained by the above screening method.

The expression-induction inhibitor of HSPs refers to those comprising as an active ingredient a compound capable of inhibiting induction of expression of HSPs by inhibiting expression of HSPs in cells mainly on a transcriptional level.

In the present invention, an expression-induction inhibitor for HSP70 is preferred, of which the relevance to thermal resistance or radiation resistance has been suggested, and particularly an expression-induction inhibitor for human HSP70 is preferred.

In addition, the present invention provides an expression inducer of HSPs, which can be obtained by the above screening method.

An expression inducer of HSPs of the present invention refers to those comprising as an active ingredient a compound capable of inducing expression of HSPs by inducing expression of HSPs in cells mainly on a transcriptional level.

In addition, the present invention provides an expression-induction enhancer of HSPs, which can be obtained by the above screening method.

The expression-induction enhancer of HSPs of the present invention refers to those comprising as an active ingredient a compound capable of enhancing induction of expression of HSPs by enhancing induction of expression of HSPs in cells mainly on a transcriptional level.

In the present invention, an expression inducer or expression-induction enhancer of HSP70 is preferred, of which the relevance to protective action against ischemia, enhancement of wound healing and protective action against ulcer has been suggested, and particularly an expression inducer or expression-induction enhancer for human HSP70 is preferred.

Concrete examples of the expression-induction inhibitor of heat shock proteins of the present invention include those comprising as an active ingredient a quinazoline derivative represented by the general formula (I) or (II): 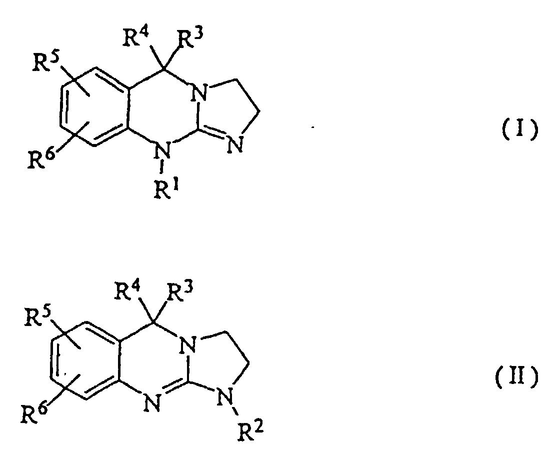 wherein R¹ is hydrogen atom, an alkyl group having 1 to 8 carbon atoms, an alkenyl group having 3 to 8 carbon atom, or an alkynyl group having 3 to 8 carbon atoms; R² is hydrogen atom, an alkyl group having 1 to 8 carbon atoms, or an aralkyl group having 7 to 10 carbon atoms; R³ is hydrogen atom or hydroxyl group; R⁴ is a substituted or unsubstituted phenyl group, or a cycloalkyl alkyl group having 5 to 8 carbon atoms; and each of R⁵ and R⁶ is hydrogen atom, a halogen, an alkyl group having 1 to 4 carbon atoms, or an alkoxy group having 1 to 4 carbon atoms.

In R¹ and R² above, the alkyl group having 1 to 8 carbon atoms includes linear alkyl groups, such as methyl group, ethyl group, propyl group, butyl group, pentyl group, hexyl group, heptyl group, and octyl group; and branched alkyl groups, such as isopropyl group, isobutyl group, *tert*-butyl group, and isopentyl group.

In R¹ above, the alkenyl group having 3 to 8 carbon atoms includes 1-propenyl group, allyl group, 2-butenyl group, 3-butenyl group, 3-pentenyl group, 4-hexenyl group, 5-heptenyl group, and 6-octenyl group.

In R¹ above, the alkynyl group having 3 to 8 carbon atoms includes 2-propynyl group, 3-butynyl group, 4-pentynyl group, 5-hexynyl group, 6-heptynyl group, and 7-octynyl group.

Among R¹ mentioned above, hydrogen atom, an alkyl group having 1 to 5 carbon atoms, or an alkenyl group having 3 to 8 carbon atoms is preferable.

In R² above, the aralkyl group having 7 to 10 carbon atoms includes benzyl group, phenethyl group, and phenylpropyl group.

Among R² mentioned above, hydrogen atom, methyl group, or benzyl group is preferable.

In R⁴ above, the substituted phenyl group includes phenyl group substituted with an alkyl group having 1 to 4 carbon atoms or with an alkoxy group having 1 to 4 carbon atoms.

In R⁴ above, the cycloalkyl alkyl group having 5 to 8 carbon atoms includes cyclobutyl methyl group, cyclopentyl methyl group, cyclohexyl methyl group, and cyclohexyl ethyl group.

Among R⁴ mentioned above, phenyl group or the cycloalkyl alkyl group having 5 to 8 carbon atoms is preferable, and phenyl group or cyclohexyl methyl group is more preferable.

In R⁵ and R⁶ above, the halogen includes fluorine atom, chlorine atom, bromine atom, and iodine atom.

In R⁵ and R⁶ above, the alkyl group having 1 to 4 carbon atoms includes linear alkyl groups, such as methyl group, ethyl group, propyl group, and butyl group; and branched alkyl groups, such as isopropyl group, isobutyl group, and *tert*-butyl group.

In R⁵ and R⁶ above, the alkoxy group having 1 to 4 carbon atoms includes methoxy group, ethoxy group, n-propoxy group, isopropoxy group, and n-butoxy group.

Among R⁵ mentioned above, hydrogen atom or the halogen is preferable, and hydrogen atom or chlorine atom is more preferable.

Among R⁶ mentioned above, hydrogen atom or the halogen is preferable, and hydrogen atom or chlorine atom is more preferable.

The positions of R⁵ and R⁶ are, but not particularly limited, preferably at 7-position and 8-position, respectively, in the back bone of the quinazoline derivative of the general formula (I) or (II).

Preferred concrete examples of the quinazoline derivative represented by the above general formula (I) or (II) are given below, but the present invention is by no means limited to these compounds. Concretely, 10-methyl-5-phenyl-2,3,5,10-tetrahydroimidazo[2,1-b]quinazoline (Code No. SR-2124), 10-(n-butyl)-7-chloro-5-cyclohexylmethyl-2,3,5,10-tetrahydroimidazo[2,1-b]quinazoline (Code No. SR-1618), 10-(2'-propenyl)-5-phenyl-2,3,5,10-tetrahydroimidazo[2,1-b]quinazoline (Code No. SR-2125), 10-isopentyl-8-chloro-5-phenyl-2,3,5,10-tetrahydroimidazo[2,1-b]quinazoline (Code No. SR-1544), 10-isopentyl-7-chloro-5-phenyl-5-hydroxy-2,3,5,10-tetrahydroimidazo[2,1-b]quinazoline (Code No. SR-2158), 5-phenyl-1,2,3,5-tetrahydroimidazo[2,1-b]quinazoline (Code No. SR-2147), 1-methyl-5-phenyl-7-chloro-1,2,3,5-tetrahydroimidazo[2,1-b]quinazoline (Code No. SR-1040), 1-benzyl-5-phenyl-7-chloro-1,2,3,5-tetrahydroimidazo[2,1-b]quinazoline (Code No. SR-2210), and the like are preferable, from the viewpoint of inhibiting the expression-induction of the heat shock proteins such as HSP70. SR-1040, SR-2124, and SR-2125 are more preferable.

The structure of 1-methyl-5-phenyl-7-chloro-1,2,3,5-tetrahydroimidazo[2,1-b]quinazoline (Code No. SR-1040) is represented by the formula (III):

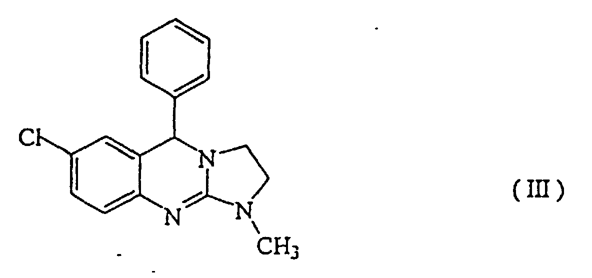

The structure of 10-(2'-propenyl)-5-phenyl-2,3,5,10-tetrahydroimidazo[2,1-b]quinazoline (Code No. SR-2125)is represented by the formula (IV):

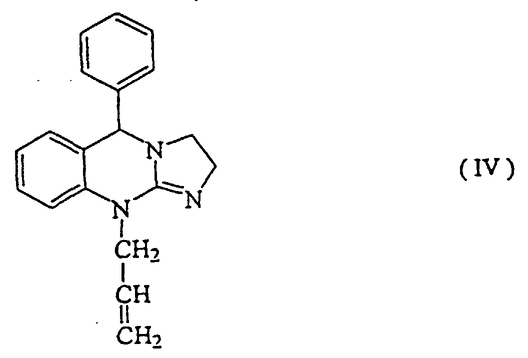

The quinazoline derivative represented by the above general formula (I) or (II) is a known compound which has been known to have pharmacological actions such as antidepression action, diuretic action, hypotensive action, and the like. Such compounds can be prepared according to the methods described in Japanese Patent Laid-Open Nos. Sho 50-116496 and Sho 54-48797.

One embodiment of the expression-induction inhibitor of heat shock proteins of the present invention comprises the above quinazoline derivative as an active ingredient. The content of the quinazoline derivative may be any amount as long as the expression-induction of the heat shock protein is inhibited. For instance, the content is preferably from 50 to 100% by weight.

The expression-induction inhibitor of heat shock proteins of the present invention may contain a pharmaceutically acceptable solvent in order to dissolve the above quinazoline derivative.

Among the above quinazoline derivatives, SR-1040 and SR-2125 inhibit the expression-induction of endogenous HSP70 as described in Example 6.

In addition, SR-1040 has an activity of inhibiting acquisition of thermotolerance in cells. The method for measurement of the inhibition activity of the acquisition of thermotolerance using the HeLa-198 strain is described in Example 7.

By the use of the expression-induction inhibitor of the heat shock proteins of the present invention, the resistance to heat or radiation can be overcome, and the cell death by heat or radiation can be enhanced, so that the effectivity of the hyperthermia or radiotherapy for cancers can be further enhanced. Further, by the inhibition of expression of P-glycoprotein, which is causative of the multiple drug resistance of cancers, the resistance in the chemotherapy for treating cancers can be overcome. As described above, the expression-induction inhibitor of the HSPs is considered to be extremely useful as carcinostatics or agents for overcoming resistance, each of which is based on a new action mechanism not conventionally found. Moreover, the expression-induction inhibitor of the HSPs is also useful in the analysis of biological control mechanism of stress-response, particularly useful in the analysis of the transcriptional regulatory mechanism which depends on stress-response, so that it is also expected in applications as reagents for research in the life science field.

The expression inducer of the heat shock proteins of the present invention, as one embodiment, comprises as an active ingredient a chalcone derivative represented by the general formula (V): 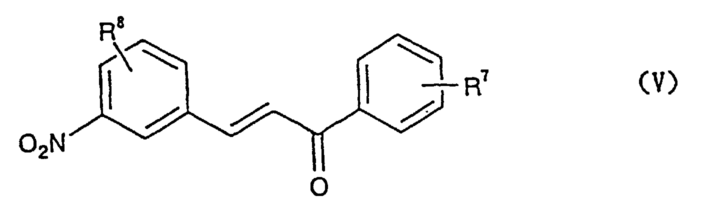 wherein R⁷ is an alkyl group having 1 to 8 carbon atoms; and R⁸ is hydrogen atom or a halogen.

In R⁷ above, the alkyl group having 1 to 8 carbon atoms includes linear alkyl groups, such as methyl group, ethyl group, propyl group, butyl group, pentyl group, hexyl group, heptyl group, and octyl group; and branched alkyl groups, such as isopropyl group, isobutyl group, *tert*-butyl group, and isopentyl group.

Among R⁷ mentioned above, an alkyl group having 1 to 4 carbon atoms is preferable.

In R⁸ above, the halogen includes fluorine atom, chlorine atom, bromine atom, and iodine atom.

Among R⁸ mentioned above, hydrogen atom or chlorine atom is preferable.

The positions of R⁷ and R⁸ are, but not particularly limited, preferably at 4-position and 2-position, respectively, owing to the orientation of the benzene ring to which each substituent is bonded.

Preferred concrete examples of the chalcone derivative represented by the above general formula are given below, but the present invention is by no means limited to these compounds. Concretely, 1-[4-(2-methylpropyl)phenyl]-3-(3-nitrophenyl)-2-propen-1-one (Code No. SR-2297), 3-(2-chloro-5-nitrophenyl)-1-(4-methylphenyl)-2-propen-1-one (Code No. SR-2607), and the like are preferable, from the viewpoint of inducing expression of the heat shock proteins such as HSP70. The SR-2297 is preferable from the viewpoint of also having activity for enhancing the expression-induction of the heat shock proteins such as HSP70.

The structure of 1-[4-(2-methylpropyl)phenyl]-3-(3-nitrophenyl)-2-propen-1-one (Code No. SR-2297) is represented by the formula (VI):

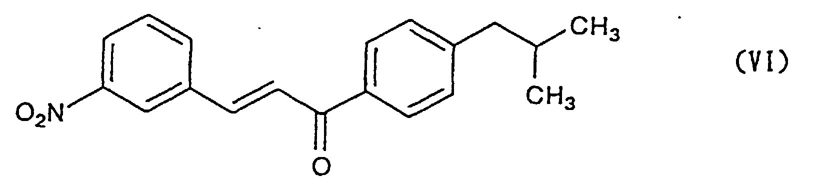

The structure of 3-(2-chloro-5-nitrophenyl)-1-(4-methylphenyl)-2-propen-1-one (Code No. SR-2607) is represented by the formula (VII):

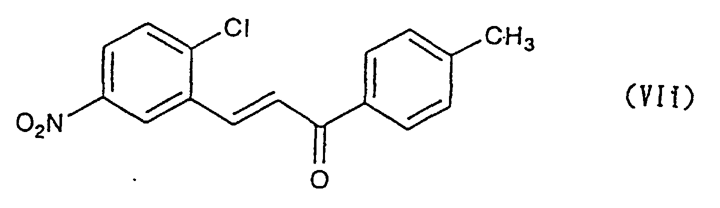

The chalcone derivative represented by the general formula (V) is a known compound, and can be prepared by conventional method.

The content of the chalcone derivative may be any amount as long as expression of the heat shock protein is induced. For instance, the content is preferably from 50 to 100% by weight.

The expression inducer of heat shock proteins of the present invention may contain a pharmaceutically acceptable solvent in order to dissolve the above chalcone derivative.

The expression-induction enhancer of heat shock proteins of the present invention includes, as one example, 1-[4-(2-methylpropyl)phenyl]-3-(3-nitrophenyl)-2-propen-1-one (Code No. SR-2297) represented by the formula (VI):

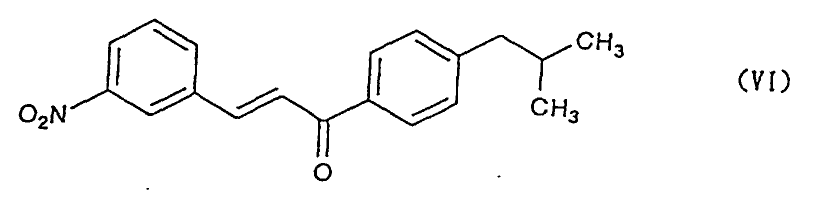

The content of the expression-induction enhancer of heat shock proteins of the present invention may be any amount as long as the expression-induction of the heat shock proteins is enhanced. For instance, the content is preferably from 50 to 100% by weight.

The expression-induction enhancer of heat shock proteins of the present invention may contain a pharmaceutically acceptable solvent in order to dissolve the above compound.

The heat shock protein expression inducer or expression-induction enhancer of the present invention exhibits an effect of acquiring ischemia resistance in neuronal cells or myocardial cells through high expression of HSP70 and the like. In addition, the expression inducer or expression-induction enhancer exhibits an effect of promoting the regeneration of a wound tissue through high expression of HSP70 and the like. Further, the expression inducer or expression-induction enhancer exhibits an effect of protecting a mucosal cell in digestive tract through high expression of HSP70 and the like. As described above, the heat shock protein expression inducer or expression-induction enhancer of the present invention is considered to be useful as a therapeutic or prophylactic agent, based on a novel action mechanism not conventionally found, against ischemic diseases, wound or ulcer. In addition, the heat shock protein expression inducer or expression-induction enhancer of the present invention is useful in the characterization of biological stress-response controlling mechanism, especially in the characterization of a transcriptional regulatory mechanism dependent on stress response, which is also expected to be useful as a reagent for research in the field of life science.

Further, the present invention provides a pharmaceutical composition useful in a cancer treatment, comprising as an active ingredient the above expression inhibitor of heat shock proteins, preferably a pharmaceutical composition useful in thermotherapy or radiotherapy against a cancer.

Since the pharmaceutical composition according to the present invention comprises as an active component the above expression-induction inhibitor of heat shock proteins, when used in hyperthermia or radiotherapy against a cancer, the pharmaceutical composition can overcome the resistance of cancer cells against heat or radiation and enhance cell death by application of hyperthermia or radiation. Therefore, the pharmaceutical composition can further enhance an effectiveness of the hyperthermia or the radiotherapy against a cancer. Further, the pharmaceutical composition can overcome the resistance in chemotherapy against a cancer by inhibiting the induction of the expression of P-glycoprotein which causes a multiple drug resistance of a cancer. As described above, the pharmaceutical composition of the present invention can serve as a carcinostatic agent or a resistance overcoming agent to treat various cancers and pre-cancer conditions.

In the pharmaceutical composition described above, there may be added a carrier, a stabilizer, an absorption accelerator and the like, as occasion demands. The resulting mixture may be administered orally as a tablet, a powder, a granule, a capsule, syrup and the like, or it may be administered parenterally as a suppository, an injection, a medicine for external application, drops and the like.

The pharmaceutical composition described above may be administered alone or together with other chemotherapeutic agents. The timing of the administration of the pharmaceutical composition when used in thermotherapy or radiotherapy against a cancer may be such that the pharmaceutical composition is administered prior to or simultaneously with such a therapy. For example, it is preferable that the pharmaceutical composition is administered within 24 hours before the therapy.

The dose of the heat shock protein expression-induction inhibitor in the pharmaceutical composition according to the present invention can be appropriately adjusted depending on the target disease, the age and body weight of the patient, and the like, and is usually 0.1 mg to 1000 mg, preferably 1 mg to 500 mg, per dose, and the number of dose is preferably determined in accordance with the purpose.

In addition, the present invention provides a pharmaceutical composition comprising the above expression inducer of heat shock proteins.

In such a pharmaceutical composition, there may be added a carrier, a stabilizer, an absorption accelerator and the like, as occasion demands. The resulting mixture may be administered orally as a tablet, a powder, a granule, a capsule, syrup and the like, or it may be administered parenterally as a suppository, an injection, a medicine for external application, drops and the like.

The pharmaceutical composition described above may be administered alone or together with other medicaments.

The dose of the expression inducer of heat shock proteins in the pharmaceutical composition can be appropriately adjusted depending on the target diseases, the age and body weight of the patient, and the like, and is usually 0.1 mg to 1000 mg, preferably 1 mg to 500 mg, per dose, and the number of dose is preferably determined appropriately in accordance with the purpose.

In addition, the present invention provides a pharmaceutical composition comprising the above expression-induction enhancer of heat shock proteins.

In such a pharmaceutical composition, there may be added a carrier, a stabilizer, an absorption accelerator and the like, as occasion demands. The resulting mixture may be administered orally as a tablet, a powder, a granule, a capsule, syrup and the like, or it may be administered parenterally as a suppository, an injection, a medicine for external application, drops and the like.

The pharmaceutical composition described above may be administered alone or together with other medicaments.

The dose of the expression-induction enhancer of heat shock proteins in the pharmaceutical composition can be appropriately adjusted depending on the target diseases, the age and body weight of the patient, and the like, and is usually 0.1 mg to 1000 mg, preferably 1 mg to 500 mg, per dose, and the number of dose is preferably determined appropriately in accordance with the purpose.

Since the pharmaceutical compositions of the present invention each comprises as an active ingredient an expression inducer or expression-induction enhancer of heat shock proteins, it is possible to treat or prevent ischemic diseases, wound or ulcer through high expression of HSP70 and the like as described above.

The present invention will be described in further detail by means of the following working examples, but the present invention is by no means limited to these working examples. Unless specified otherwise, the following examples were carried out by methods described in Sambrook, J. et al., *Molecular Cloning: A Laboratory Manual, 2nd edition,* Cold Spring Harbor Laboratory, New York, published in 1989; edited by Ausubel, F. M. et al., *Current Protocols in Molecular Biology,* John Wiley and Sons, Inc., and the like.

### Example 1

### Construction of Plasmids

A reporter plasmid pHBCAT [Wu, B. et al. (1985), *Mol. Cell Biol.* **5**, 330-341; Wu, B. et al. (1986) *Proc. Natl. Acad. Sci. USA* **83**, 629-633] obtained by ligating 5'-upstream region 2.8 kb of a human HSP70 gene to a chloramphenicol acetyl transferase (CAT) gene was digested with *Bam*HI. The digested product was then subjected to agarose gel electrophoresis, and a 2.8 kb band was extracted from the gel, to prepare a *Bam*HI fragment containing the 5'-upstream region of the human HSP70 gene. The fragment thus obtained was inserted into the *Bgl*II site of pGL2-Basic (manufactured by Promega) to prepare a reporter plasmid pH70LUC-85 in which a firefly luciferase gene was ligated to the 5'-upstream region, 2.8 kb, of the human HSP70 gene.

In order to examine whether or not luciferase was induced from the reporter plasmid pH707LUC-85 described above in response to heat shock, the luciferase activity was confirmed in a transient expression system using HeLa cells. Specifically, HeLa cells were inoculated at 1.0 × 10⁴ cells/cm² in a 10 cm culture dish and cultured in DMEM containing 10% FCS at 37°C under a 5%-CO₂ atmosphere for 24 hours, and thereafter transformed with 10 µg of pH70LUC-85 and 4 µg of pSVβ-Galactosidase (manufactured by Promega) by a DEAE-dextran method. After culturing for 48 hours, heat shock at 41° or 42°C for 2 hours was applied to the resulting cells followed by recovery-culturing at 37°C for 2 hours. After washing the surface of the cells with PBS, the cells were scraped using a cell scraper. The scraped cells were collected by centrifugation, and the luciferase activity was examined in the cell extracts according to the protocol by luciferase assay system (manufactured by Promega) with a luminometer ("LUMINOSKAN," manufactured by Labsystems).

As a result, it was found that while the control cell extract without applying heat shock detected no luciferase activity, 4.9 RLU (Relative Light Units) and 2.5 RLU of luciferase activities were detected in the cell extracts with heat shock at 41° and 42°C, respectively, indicating that luciferase was induced from pH70LUC-85 in response to the heat shock.

Subsequently, a neomycin resistant gene (*neo*) was incorporated into the pH70LUC-85 described above. Specifically, pBluescript-*pgk-neo* in which a *pgk-neo* cassette [McBurney, M. W. et al., (1991) *Nucleic Acids Res.* **20**, 5755-5761) was inserted into the *Eco*RI-*Hind*III site in pBluescript IISK+ (manufactured by Stratagene) was digested with *Not*I and ligated to an *Sal*I-*Not*I adapter. Thereafter, the ligated product was digested with *Sal*I, to prepare a 2 kb *pgk-neo* fragment of which both terminals were *Sal*I cohesive ends. The *pgk-neo* fragment thus obtained was ligated to the *Sal*I site just downstream of the 3'-non-translational region of the luciferase gene in pH70LUC-85, to prepare a reporter plasmid pHLUC-*neo* harboring a neomycin resistant gene.

### Example 2

### Establishment of Cell Line

Using 10 µg of pHLUC-*neo* obtained in Example 1, the 5 × 10⁶ Hela cells (ATCC CCL2) were transformed by a conventional electroporation method (voltage: 250 V, charge: 25 µF, pulse: 2 times). After the electroporation, the transformed cells were cultured in DMEM containing 700 µg/ml of G-418 and 10% FCS at 37°C under a 5%-atmosphere, whereby selecting G-418 resistant transformants. The transformants thus obtained were grown, and the heat shock-dependent luciferase activities were examined at 90% confluent in 10 cm culture dishes.

Specifically, heat shock at 42°C for 2 hours was applied to the G-418 resistant transformants described above, and then subjected to recovering culture at 37°C for 2 hours. Thereafter, the luciferase activity in a cell extract was determined with a luminometer in the same manner as in Example 1.

When 30 clones of the G-418 resistant transformants were examined for the luciferase activity, the induction of the heat shock-dependent luciferase activity was observed in 6 clones as shown in Table 1.

**Table 1**

| Clone | Luciferase Activity (RLU) | | Fold Induction |
|---|---|---|---|
| | at 37°C | at 42°C | |
| HeLa-196 | 113 | 4,160 | 36.8 |
| HeLa-197 | 0.19 | 216 | 1,140 |
| HeLa-198 | 250 | 98,100 | 392 |
| HeLa-199 | 75.5 | 639 | 8.46 |
| HeLa-201 | 63.5 | 3,320 | 52.3 |
| HeLa-204 | 1,880 | 19,400 | 10.3 |

Among these clones, the HeLa-198 strain which showed high fold induction of luciferase by heat shock and gave a high level of expression to an extent sufficient to detect the luciferase activity even in the assay system using a 96-well culture plate was considered to be most suitable to the screening for an HSP70 expression-induction controlling substance of the present invention.

### Example 3

### Responsiveness of HeLa-198 Strain to HSP70 Expression Inducer

Using quercetin, known to have an inhibitory activity of HSP70 expression-induction, there was examined whether or not the induction of the heat shock-dependent luciferase in the HeLa-198 strain obtained in Example 2 was inhibited. The HeLa-198 strain was inoculated in a 96-well culture plate at 3.0 × 10³ cells/well and cultured at 37°C under a 5%-CO₂ atmosphere for 48 hours. Thereafter, quercetin (manufactured by Sigma) was added so as to have a final concentration of 50 to 200 µM, and then the culture was continued for additional 1 hour at 37°C. Subsequently, the heat shock at 42°C for 2 hours was applied, and then the recovering culture was performed at 37°C for 2 hours. As a result of measuring the luciferase activity of these cells, it was found that the heat shock-induced luciferase activity was suppressed by quercetin at 50 µM or higher. It was confirmed by MTT [3-(4,5-dimethylthiazol-2-yl)-2,5-diphenyltetrazolium bromide] assay using Cell Proliferation Kit (manufactured by Boehringer Mannheim) that this effect did not result from any damages of the cells by the action of quercetin. In addition, quercetin had no effect on expression of luciferase on a background level when no heat shock was applied.

It was found from the results described above that the HeLa-198 strain could be used for the screening for an HSP70 expression-induction inhibitor.

### Example 4

### Responsiveness of HeLa-198 Strain to HSP70 Expression Inducer

Prostaglandin A1 (PGA1) has been known to have an HSP70 expression-induction activity (Amici, C. at al. (1992) *Proc. Natl. Acad. Sci. USA* **91**, 2280-2284]. To a culture of the HeLa-198 strain was supplemented 50 µM of PGA1 and cultured at 37°C under a 5%-CO₂ atmosphere for 5 hours, and the luciferase activity in the cell extract was then examined. As a result, the luciferase activity was revealed to be higher by two to three times when compared with the control without addition of PGA1. From the above, it was revealed that the HeLa-198 strain could be used for the screening for an HSP70 expression inducer.

### Example 5

### Method of Screening HSP70 Expression Induction Controlling Substance

As a compound to be tested, each of the compounds shown in Table 2 was prepared by methods described in Japanese Patent Laid-Open Nos. Sho 50-116496 and Sho 54-48797. The HeLa-198 strain obtained in Example 2 was inoculated in a 96-well culture plate at 3.0 × 10³ cells/well and cultured in DMEM at 37°C under a 5%-CO₂ atmosphere for 48 hours. The culture medium was replaced with DMEM containing a compound to be tested dissolved in DMSO so as to have a final concentration of 10 to 50 µM, and thereafter the culture was carried out at 37°C for additional 1 hour under a 5%-CO₂ atmosphere. Subsequently, the heat shock at 42°C for 2 hours was applied, and the recovering culture was then performed at 37°C for 2 hours. After removal of the medium, the luciferase activity in the cell extract was determined with a luminometer ("LUMINOSKAN," manufactured by Labsystems) according to the protocol of Luciferase Assay System (manufactured by Promega). The luciferase activity (X) in the cells treated with a compound to be tested was obtained, wherein the luciferase activity induced in the cells treated with DMEM containing only the solvent DMSO was regarded as 100. The difference thereof (100-X) is indicated in Table 2 as an inhibition ratio of luciferase induction.

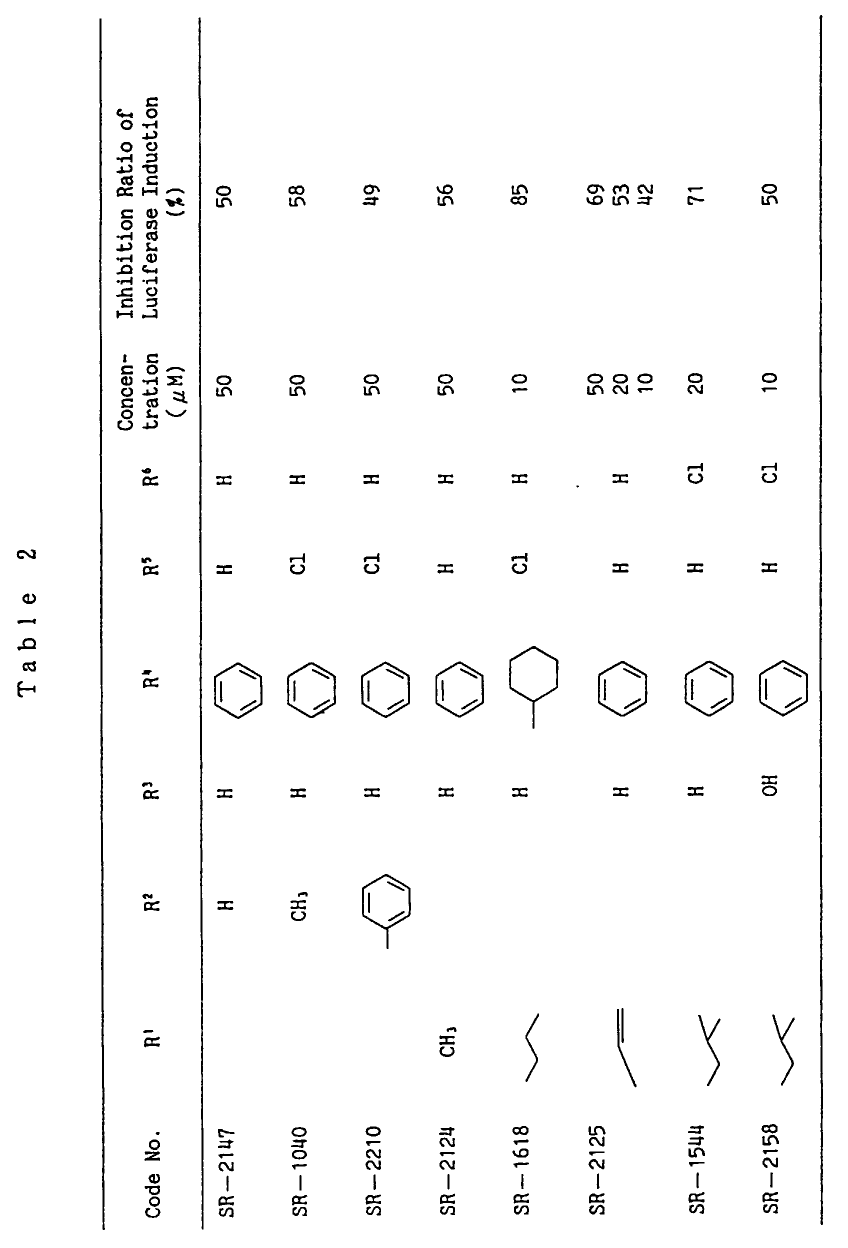

As shown in Table 2, all compounds to be tested including SR-1040 and SR-2125 inhibited the luciferase induction. Also, SR-2125 inhibited the luciferase induction in a concentration-dependent manner.

Subsequently, an SV40 early promoter was selected in order to examine whether or not SR-1040 served to act on the transcription from a promoter other than that of HSP70. First, the *pgk-neo* cassette described above was incorporated into the *Sal*I site of a luciferase expression plasmid PGL2-Control (manufactured by Promega) utilizing the SV-40 early promoter to prepare pSVLUC-*neo*. Subsequently, pSVLUC-*neo* was used to transform HeLa cells as described above, and a cell line HeLa-SV-3, which expressed luciferase under the control of the SV40 early promoter, was established.

To a culture of the HeLa-SV-3 cells was supplemented SR-1040 (final concentration: 50 µM) and cultured for additional 5 hours, and thereafter the luciferase activity in the cell extract was examined in the same manner as in Example 1. As a result, no significant difference was found, as compared to the control without addition of SR-1040 (with addition of only the solvent DMSO). Accordingly, the inhibition of the luciferase expression with the SR-1040 was considered to be HSP promoter-specific.

### Example 6

### Northern Blotting and Western Blotting

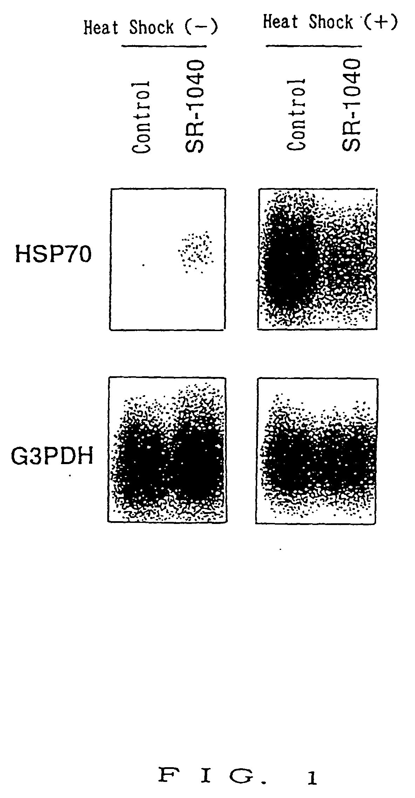

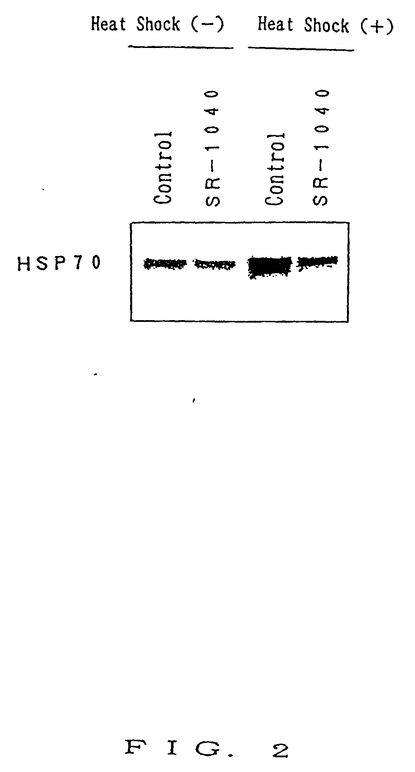

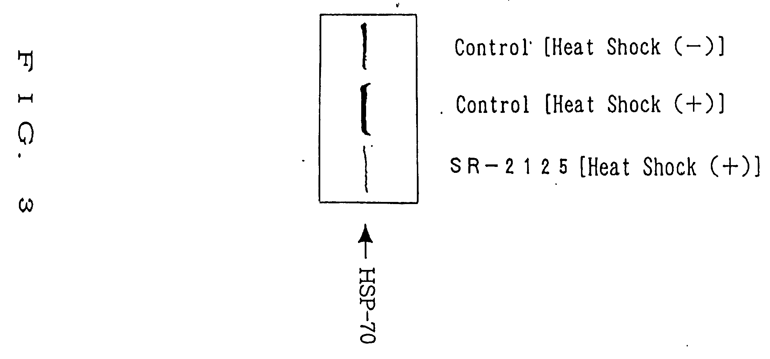

The action of SR-1040 on the induction of expression of endogenous HSP70 was studied on the mRNA level and the protein level by means of Northern blotting and Western blotting, respectively. The action of SR-2125 on the induction of expression of endogenous HSP70 was also studied on the protein level in the same manner by means of Western blotting. The HeLa-198 strain was inoculated in a 10 cm culture dish at 1.1 × 10⁶ cells/cm² and cultured for 48 hours in the same manner as in Example 5, and then a solution of SR-1040 or SR-2125 of Example 5 was supplemented so as to have a final concentration of 50 µM. Thereafter, the mixture was cultured at 37°C for additional 1 hour. Immediately after applying heat shock at 42°C for 2 hours, a whole RNA was prepared by AGPC (acidic guanidium-phenol-chloroform) method, and the resulting whole RNA was subjected to Northern blotting to detect HSP70 mRNA and an internal standard glyceraldehyde-3-phosphate dehydrogenase (G3PDH) mRNA (Figure 1). In addition, after applying heat shock at 42°C for 2 hours followed by recovery-culturing at 37°C for 2 hours, the resulting cell extract was subjected to SDS-polyacrylamide gel electrophoresis followed by Western blotting using an anti-HSP70 antibody (manufactured by StressGen) to quantity the intracellular HSP70 accumulation (Figure 2 and Figure 3).

As shown in Figure 1 and Figure 2, it was confirmed on the mRNA level and the protein level that SR-1040 inhibited the induction of expression of endogenous HSP70 by applying heat shock at 42°C for 2 hours. Also, as shown in Figure 3, it was confirmed on the protein level that SR-2125 similarly inhibited the induction of expression of endogenous HSP70 by applying heat shock at 42°C for 2 hours.

### Example 7

### Inhibitory Activity on Acquisition of Thermotolerance in Cells

There was examined whether or not a quinazoline derivative comprising SR-1040 having an inhibitory activity of HSP70 expression-induction as studied in Example 5 inhibited the acquisition of thermotolerance in cells.

While the HeLa-198 strain undergoes a morphological change when exposed to heat shock at 45°C for 50 minutes and is detached from the bottom of a culture flask, leading to death, it has been found that the cells which have been subjected previously to non-lethal heat shock at 42°C for 2 hours does not undergo morphological changes even when 6 hours thereafter lethal heat shock at 45°C for 50 minutes was applied, so that it has been found that the cells acquired thermotolerance. Here, when SR-1040 (final concentration: 50 µM) was acted 1 hour prior to the non-lethal heat shock described above, the number of living cells after applying the heat shock at 45°C for 50 minutes was markedly reduced, causing almost all cells to be detached from the bottom of the flask and observed as dead cells in a manner similar to the case where lethal heat shock was directly applied at 45°C for 50 minutes. In this procedure, when SR-1040 was acted only during the lethal heat shock at 45°C for 50 minutes, no morphological changes were found in the cells, so that the acquisition of thermotolerance was observed. These findings suggest that the presence of SR-1040 during the non-lethal heat shock at 42°C for 2 hours inhibits the induction of the HSP70 expression, whereby the thermotolerance could not consequently be acquired by HeLa-198 cells.

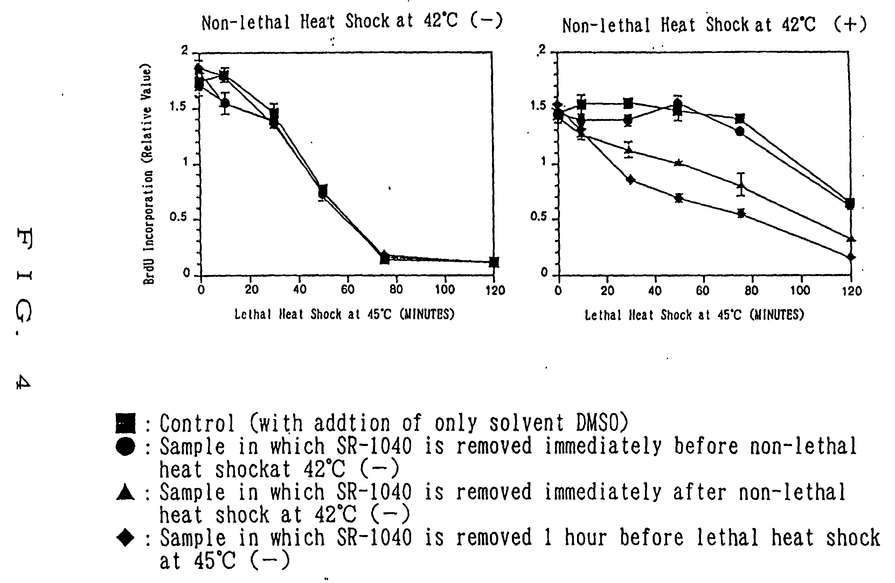

Subsequently, for the purpose of ensuring further objectivity and quantitativeness with regard to inhibitory activity of the acquisition of thermotolerance described above, the inhibitory activity of the acquisition of thermotolerance was assayed using cell proliferation potency by measuring, as an index, incorporation of bromodeoxyuridine (BrdU), a thymidine analogue. SR-1040 was supplemented so as to have a final concentration of 50 µM, and after 1 hour, non-lethal heat shock (42°C for 2 hours) was applied to the HeLa-198 strain followed by recovery-culturing at 37°C for 6 hours, further followed by applying lethal heat shock at 45°C for 10 to 120 minutes. During this course, the culture medium was replaced with an SR-1040-free medium immediately before the non-lethal heat shock, immediately after the non-lethal heat shock, or 1 hour before the lethal heat shock, to remove the SR-1040 from the culture system. After the lethal heat shock, recovering culture at 37°C for 18 hours was carried out, and then the culture was continued for 6 hours, with supplementing BrdU in accordance with the protocol of a cell proliferation assay system ("Cell proliferation ELISA system" manufactured by Amersham). BrdU incorporation by the cells was quantified in accordance with the protocol (Figure 4).

Figure 4 shows the cell proliferation potency in the case of applying the lethal heat shock at 45°C as described above. As shown in Figure 4, in the case where no non-lethal heat shock at 42°C for 2 hours was applied, a similar proliferation potency was observed regardless of the presence or absence of SR-1040. On the other hand, in the case where SR-1040 was absent when the non-lethal heat shock was applied, almost no reduction in the cell proliferation potency was observed even when the heat shock at 45°C was applied for 75 minutes, so that it was found that the cell acquired the thermotolerance. However, in the case where SR-1040 was present when the non-lethal heat shock was applied, a marked reduction in the cell proliferation potency was observed by applying the heat shock at 45°C, so that it has been found that the acquisition of thermotolerance in the HeLa-198 strain was inhibited.

### Example 8

### Reporter Gene Expression Induction Activity of SR-2297

A chemical library comprising SR-2297 and related compounds thereof was screened as described in Example 5, and it was found that SR-2297 at 10 µM gave an increase of about three times in expression of the luciferase activity in the HeLa-198 strain. Specifically, the HeLa-198 strain obtained in Example 2 was inoculated in a 96-well culture plate at 3.0 × 10³ cells/well and cultured in DMEM containing 10% FCS at 37°C under a 5%-CO₂ atmosphere for 48 hours. The culture medium was replaced with a medium containing 10 µM of SR-2297 (DMEM containing 0.1% of a 10 mM solution of SR-2297 dissolved in DMSO), and the culture was carried out at 37°C for additional 5 hours under a 5%-CO₂ atmosphere. Subsequently, the medium was removed, and the luciferase activity in the cell extract was determined with a luminometer ("LUMINOSKAN," manufactured by Labsystems) according to the protocol of Luciferase Assay System (manufactured by Promega).

As a result, while the luciferase activity of 2.33 RLU was detected in the cells cultured in a medium containing only the solvent DMSO, the luciferase activity of the cells cultured in the medium containing SR-2297 was 6.97 RLU.

### Example 9

### Time Course of Induction of Reporter Gene Expression by SR-2297

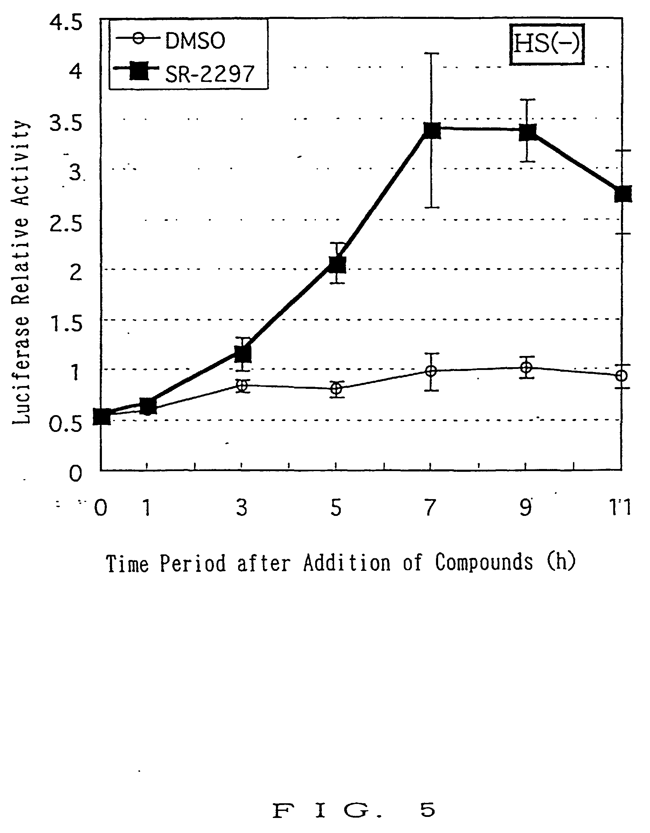

Subsequently, the induction of the luciferase activity was followed with the time course when the HeLa-198 strain was cultured in a medium containing SR-2297. The HeLa-198 strain was cultured in the same manner as in Example 8, and the intracellular luciferase activity was determined at points 1, 3, 5, 7, 9 and 11 hours after addition of SR-2297 (Figure 5).

As shown in Figure 5, SR-2297 exhibited luciferase induction activity higher by 2 to 3.5 times, as compared to that of the control with DMSO treatment over the time period ranging from 5 to 11 hours after the addition.

### Example 10

### Enhancing Activity on Reporter Gene Expression-Induction of SR-2297

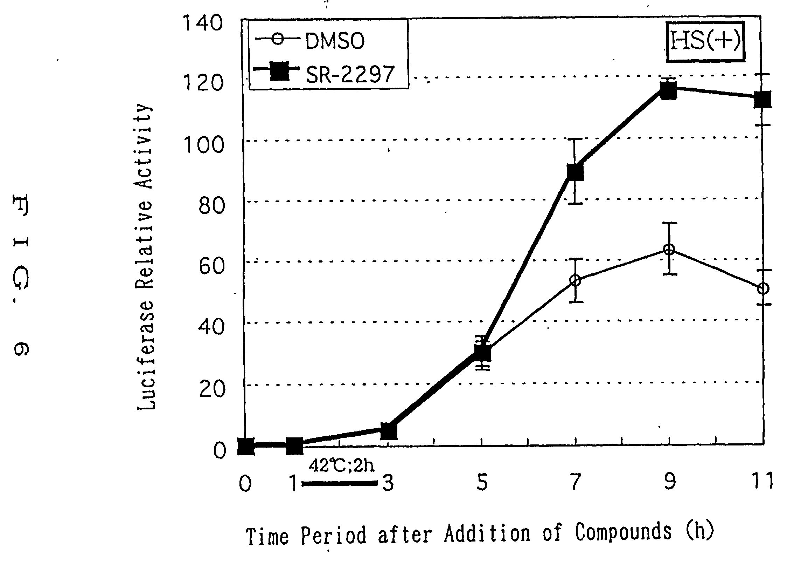

There was examined whether or not SR-2297 further enhanced luciferase induction by heat shock. The HeLa-198 strain was inoculated in a 96-well culture plate at 3.0 × 10³ cells/well and cultured in the same manner as in Example 8. The culture medium was replaced with a medium containing 10 µM of SR-2297, and the cells were cultured for additional 1 hour at 37°C. Subsequently, heat shock was applied to the resulting cells at 42°C for 2 hours, and thereafter the resulting cells were subjected to recovering culture at 37°C for 2 hours. The medium was removed at points 1, 3, 5, 7, 9 and 11 hours after replacement of the culture medium with a medium containing 10 µM of SR-2297, and the luciferase activity in the cell extract was determined. As a control, the same treatment was carried out in a medium containing only DMSO (Figure 6).

As shown in Figure 6, the induction of luciferase activity after heat shock increased to about 2 times in the presence of SR-2297 for a time period ranging from 7 to 11 hours (4 to 8 hours after applying heat shock), and it was found that SR-2297 is also an expression-induction enhancer for heat shock.

### Example 11

### Endogenous HSP70 Expression-Induction Activity of SR-2297

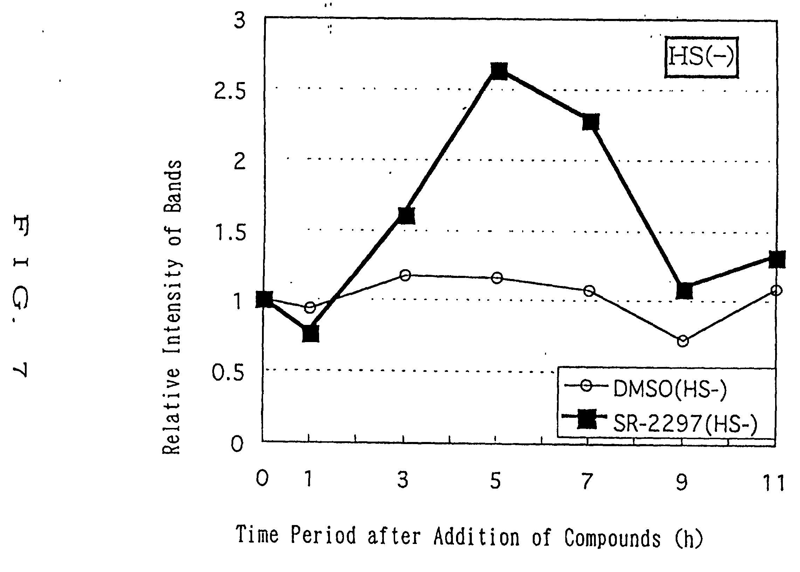

The action of SR-2297 on the induction of endogenous HSP70 expression was studied by Western blotting. The HeLa-198 strain was inoculated in a 10 cm culture dish at 1.1 × 10⁶ cells/cm² and cultured for 48 hours. Thereafter, to the resulting culture was supplemented SR-2297 so as to give a final concentration of 10 µM. The culture was continued at 37°C, and cell extracts were prepared 1, 3, 5, 7, 9 and 11 hours after addition of SR-2297. Each cell extract was subjected to SDS-polyacrylamide gel electrophoresis followed by Western blotting using an anti-HSP70 antibody (manufactured by StressGen) to quantify the intracellular HSP70 accumulation. The intensity of the bands obtained by Western blotting was quantified with Bioimage Analyzer (manufactured by Fuji Photo Film Co., Ltd.), and indicated as numeric data. As a control, a medium containing only the DMSO was subjected to a similar treatment (Figure 7).

As shown in Figure 7, it was found that SR-2297 exhibited a marked induction action on the expression of endogenous HSP70.

### Example 12

### Endogenous HSP70 Expression-Induction Enhancing Activity of SR-2297 by Heat Shock

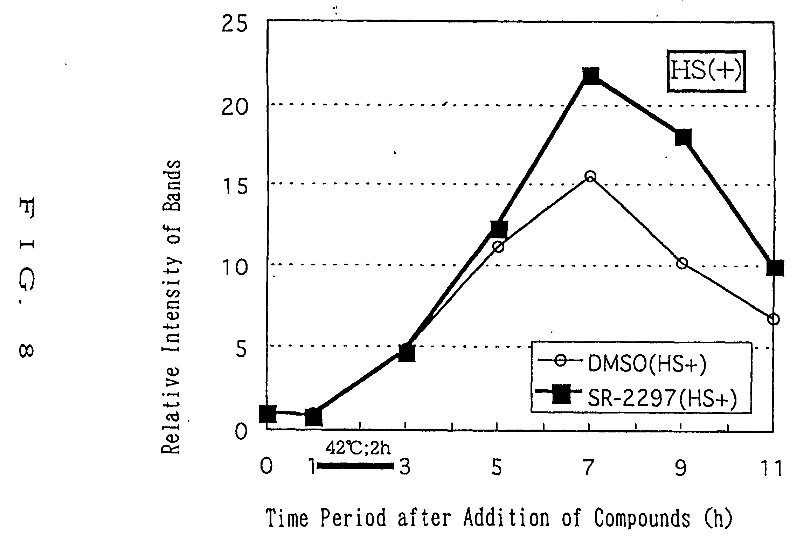

The action of SR-2297 on the induction of endogenous HSP70 expression by heat shock was studied in the same manner as in Example 11. The HeLa-198 strain was cultured in a 10 cm culture dish for 48 hours, and then the culture medium was replaced with a medium containing 10 µM of SR-2297, and the culture was carried out at 37°C for additional 1 hour. Subsequently, the cells were subjected to heat shock at 42°C for 2 hours followed by recovery-culturing at 37°C. Cell extracts were prepared at points 1, 3, 5, 7, 9 and 11 hours after the replacement with the medium containing SR-2297, and subjected to SDS-polyacrylamide gel electrophoresis. Thereafter, Western blotting was performed using an anti-HSP70 antibody (manufactured by StressGen) to quantify the amounts of intracellular HSP70 accumulation (Figure 8).

As shown in Figure 8, it was found that SR-2297 showed a marked enhancing effect on the induction of expression of endogenous HSP70 by heat shock at 42°C for 2 hours.

### Example 13

### Reporter Gene Expression-Induction Activity of SR-2607

Luciferase induction activity of an SR-2297 analog compound, SR-2607, and related compounds thereof was examined in the same manner as in Example 8. The cell medium of HeLa-198 strain was replaced with a medium containing 10 µM each of one compound, and the resulting HeLa-198 strain was cultured at 37°C for 5 hours. Thereafter, luciferase activity in the cell extract was determined.

As a result, while luciferase activity of 4.50 RLU was detected in the cells where solvent DMSO only was added, the luciferase activity of the cells cultured in a medium containing SR-2607 was 10.47 RLU, SR-2607 (10 µM) increasing the luciferase activity of the HeLa-198 strain by about 2.3 times.

### INDUSTRIAL APPLICABILITY

According to the present invention, there can be provided a method of screening a controlling substance for induction of expression of heat shock proteins; an expression-induction inhibitor, an expression inducer and an expression-induction enhancer of heat shock proteins, each obtainable by such a method; and a pharmaceutical composition comprising one of the expression-induction inhibitor, the expression inducer and the expression-induction enhancer of heat shock protein. By carrying out the method of screening of the present invention, it is possible to easily select a controlling substance for induction of expression of heat shock proteins, and to clarify or treat the pathology by various biological stress responses concerned with heat shock proteins.

## Claims

1. A method of screening a substance for controlling induction of expression of a heat shock protein, comprising the step of examining expression of reporter gene products in the presence of a compound to be tested by using a cell line transformed with a vector in which a reporter gene is ligated to downstream of a promoter of a heat shock protein gene.

2. The method of screening according to claim 1, wherein said heat shock protein gene is a gene of heat shock protein having a molecular weight of 70 kilo daltons (HSP70).

3. The method of screening according to claim 1 or 2, wherein said reporter gene is a luciferase gene.

4. The method of screening according to any one of claims 1 to 3, wherein said cell line is HeLa cells.

5. The method of screening according to any one of claims 1 to 4, wherein said substance for controlling induction of expression is an expression-induction inhibitor, an expression inducer or an expression-induction enhancer of a heat shock protein.

6. An expression-induction inhibitor of a heat shock protein obtainable by a screening method comprising the step of examining inhibition of expression of reporter gene products in the presence of a compound to be tested by using a cell line transformed with a vector in which a reporter gene is ligated to downstream of a promoter of a heat shock protein gene.

7. An expression inducer of a heat shock protein obtainable by a screening method comprising the step of examining induction of expression of reporter gene products in the presence of a compound to be tested by using a cell line transformed with a vector in which a reporter gene is ligated to downstream of a promoter of a heat shock protein gene.

8. An expression-induction enhancer of a heat shock protein obtainable by a screening method comprising the step of examining enhancement of expression of reporter gene products in the presence of a compound to be tested by using a cell line transformed with a vector in which a reporter gene is ligated to downstream of a promoter of a heat shock protein gene.

9. A pharmaceutical composition comprising the expression-induction inhibitor of a heat shock protein according to claim 6 as an active ingredient.

10. A pharmaceutical composition comprising the expression inducer of a heat shock protein according to claim 7 as an active ingredient.

11. A pharmaceutical composition comprising the expression-induction enhancer of a heat shock protein according to claim 8 as an active ingredient.

12. Use of the expression-induction inhibitor according to claim 6 for the preparation of a pharmaceutical composition for the treatment of cancer.

13. Use of the expression inducer according to claim 7 or the enhancer for expression-induction according to claim 8 for the preparation of a pharmaceutical composition for the treatment of ischemic disease, wound or ulcer.
